(19) Europäisches Patentamt
European Patent Office
Office européen des brevets

(11) Numéro de publication : 0 309 324 B1

(12) # FASCICULE DE BREVET EUROPEEN

(45) Date de publication du fascicule du brevet : 13.03.91 Bulletin 91/11

(51) Int. Cl.$^5$ : C07C 227/18, C07C 229/24, C07D 209/42

(21) Numéro de dépôt : 88402340.9

(22) Date de dépôt : 16.09.88

(54) **Procédé de synthèse d'alpha amino N alkyles et de leurs esters. Application à la synthèse de carboxyalkyl dipeptides.**

(30) Priorité : 17.09.87 FR 8712902

(43) Date de publication de la demande : 29.03.89 Bulletin 89/13

(45) Mention de la délivrance du brevet : 13.03.91 Bulletin 91/11

(84) Etats contractants désignés : AT BE CH DE ES FR GB GR IT LI LU NL SE

(56) Documents cités :
EP-A- 0 049 658
EP-A- 0 095 163
EP-A- 0 308 341
US-A- 4 296 110
ALDRICH, France, 1986-1987; p. 275

(73) Titulaire : ADIR ET COMPAGNIE
22, rue Garnier
F-92201 Neuilly sur Seine (FR)

(72) Inventeur : Vincent, Michel
8 allée du Prunier Hardy
F-92220 Bagneux (FR)
Inventeur : Baliarda, Jean
25 avenue Jeanne d'Arc
F-92160 Anthony (FR)
Inventeur : Marchand, Bernard
71 rue Laveau
F-45430 Checy (FR)
Inventeur : Remond, Georges
9 avenue des Etats-Unis
F-78000 Versailles (FR)

EP 0 309 324 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de synthèse industrielle d' α amino diacides N-alkylés, éventuellement estérifiés et leur application à la synthèse industrielle de carboxyalkyl dipeptides.

Plus spécifiquement, la présente invention concerne un nouveau procédé de synthèse industrielle de dérivés de formule générale (I) :

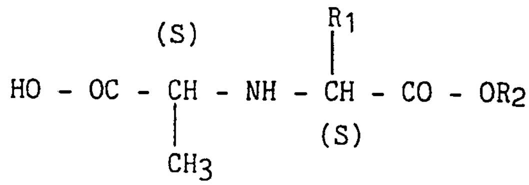

(I)

et leurs sels d'addition à un acide ou une base minéral organique,
dans laquelle :

$R_1$ est alcoyle inférieur (de 1 à 6 atomes de carbone) linéaire ou ramifié,
$R_2$ est de l'hydrogène ou un groupement alcoyle inférieur (de 1 à 4 atomes de carbone) linéaire ou ramifié,
et les deux atomes de carbone asymétriques ont la configuration (S).

Les dérivés de formule (I) obtenus selon le procédé de l'invention sont utiles dans la synthèse de carboxy alkyl dipeptides de formule (II) :

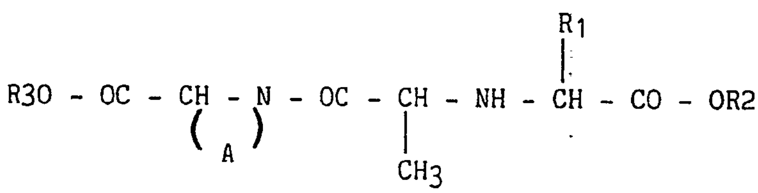

(II)

ainsi que dans celle de leurs sels pharmaceutiquement acceptables,
dans laquelle :

- $R_1$ et $R_2$ ont la même signification que dans la formule (I),
- $R_3$ est un atome d'hydrogène ou groupement alcoyle inférieur de 1 à 4 atomes de carbone
- La structure

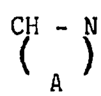

représente l'indoline, l'isoindoline, la tétrahydroquinoléine, la tétrahydroisoquinoléine, le perhydroindole, le perhydroisoindole, la perhydroisoquinoléine, la perhydroquinoléine, le perhydrocyclopenta [b] pyrrole, l'aza-2-bicyclo [2,2,2] octane, l'aza-2 bicyclo [2,2,1] heptane.

Le composé de formule (II) préféré est le perindopril de formule (III)

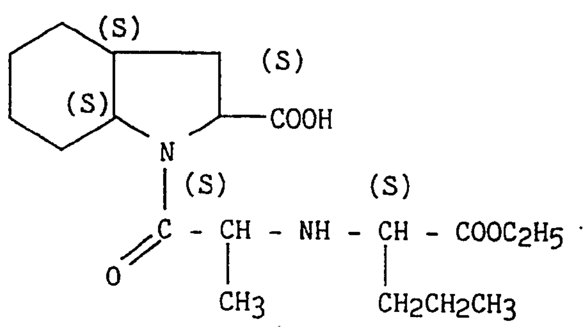

(III)

ou acide {[(éthoxycarbonyl)-1 butylamino-(S)]-2 propionyl-(S)}-1 octahydroindole carboxylique-2 (2S, 3aS, 7aS),

ainsi que ses sels d'addition à un acide ou une base pharmaceutiquement acceptables,
pour lequel le procédé de la présente invention peut être plus particulièrement appliqué.

Les composés de formule (II) ainsi que leurs sels possèdent des propriétés pharmacologiques intéressantes. Ils exercent notamment une activité inhibitrice sur certaines enzymes, comme les carboxypolypeptidases, les enképhalinases ou la kininase II. Ils inhibent notamment la transformation du décapeptide angiotensine I en l'octapeptide angiotensine II, responsables dans certains cas de l'hypertension artérielle, en agissant sur l'enzyme de conversion.

L'emploi en thérapeutique de ces composés permet donc de réduire ou même supprimer l'activité de ces enzymes responsables de la maladie hypertensive ou de l'insuffisance cardiaque. L'action sur la kininase II a pour résultat l'augmentation de la bradykinine circulante et également la baisse de la tension artérielle par cette voie.

Des composés de formule (II) et, plus particulièrement le composé de formule (III), sa préparation et son utilisation en thérapeutique ont été décrits dans le brevet européen n° 0 049 658.

On peut utiliser les composés de formule (I) pour la préparation des composés de formule (II).

Les composés de formule (I) comprennent deux carbones dits asymétriques pouvant chacun avoir deux configurations R ou S :

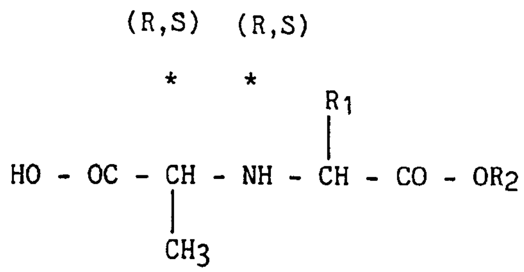

(I)

Les composés de formule (I) existent donc sous forme de quatre stéréoisomères que l'on peut désigner par (R, R) ; (R, S) ; (S, R) ou (S, S) selon la configuration des deux carbones dits asymétriques.

Or, les composés de formule (II) les plus actifs sont ceux pour lesquels les deux carbones de la chaîne latérale ont tous deux la configuration S.

C'est la raison pour laquelle le procédé selon la présente invention s'intéresse plus particulièrement à la synthèse industrielle des composés de formule (I) dans lesquels les deux carbones asymétriques ont tous deux la configuration S.

Peu de procédés de synthèse industrielle spécifiques des dérivés de formule (I) ont été décrits. On connaît la demande de brevet européen n° 0 117 488, très générale, qui utilise des trifluorométhane sulfonates α carboxylés. Toutefois, la stéréochimie des deux produits de départ doit être rigoureusement choisie afin d'obtenir le diastéréoisomère voulu du produit de formule (I). Les préparations 6 à 8 de la demande de brevet EP 0095163 permettent d'obtenir des composés structuralement proches des dérivés de formule (I). Toutefois les conditions opératoires décrites dans cette demande sont difficilement compatibles avec l'obtention de quantités importantes de dérivés de formule (I). Enfin le brevet US 4296110 donne des conditions générales de synthèse de dérivés de formule I sans indiquer la stéréochimie des atomes de carbone asymétriques ni donner de solution aux problèmes que rencontre l'homme de métier pour obtenir un diastéroisomère spécifique avec un rendement élevé.

La demanderesse a présentement découvert un procédé de synthèse industrielle des dérivés de formule (I) particulièrement intéressant car d'une part, très simple à mettre en oeuvre, d'autre part utilisant une matière première naturelle l'alanine, donc peu coûteuse et introduisant ainsi un élément de la chaîne possédant préalablement la configuration S.

En fin de synthèse, les isomères (S, S) et (R, S) obtenus sont séparés par une simple cristallisation fractionnée en présence d'acide maléique.

L'ensemble de ces choix, effectués après une sélection rigoureuse, permet d'obtenir une synthèse des composés de formule (I) particulièrement intéressante à appliquer industriellement. Plus particulièrement, le procédé selon l'invention consiste à utiliser comme matière première la L-Alanine de formule (IV) :

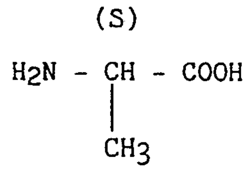

(IV)

dans laquelle :

le carbone asymétrique a la configuration <u>S</u> dont on protège le groupement carboxylique par estérification par l'alcool benzylique, en présence d'acide paratoluène sulfonique en distillant l'eau formée au fur et à mesure, pour obtenir un dérivé de formule (V) :

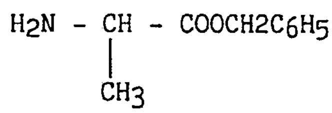

(V)

sous forme de paratoluène sulfonate que l'on transforme en la base correspondante par alcalinisation par l'ammoniaque laquelle, après extraction est condensée, sous chauffage en présence d'une amine tertiaire avec un dérivé bromé de formule (VI) :

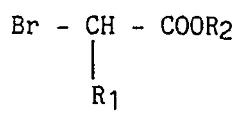

(VI)

dans laquelle, $R_1$ et $R_2$ ont la même signification que dans la formule (I), pour obtenir un dérivé de formule (VII):

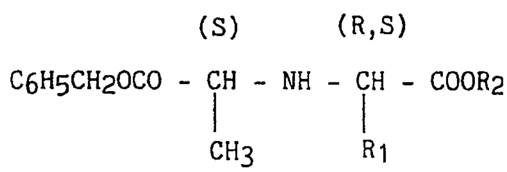

(VII)

dans laquelle, $R_1$ et $R_2$ ont la même signification que dans la formule (I),

dont on isole l'isomère (S, S) par mise en solution organique et addition d'acide maléique,

le précipité obtenu étant filtré, séché et recristallisé pour donner un maléate de l'isomère (S, S) du dérivé de formule (VII),

celui-ci étant libéré de son sel par alcalinisation d'une solution aqueuse suivie d'extraction et évaporation du solvant,

l'isomère (S, S) ainsi obtenu étant soumis à hydrogénolyse en présence de charbon palladié pour donner le composé de formule (I).

L'invention s'étend également aux produits originaux obtenus au cours de la réalisation du procédé selon l'invention, et plus particulièrement, aux sels de polyacides carboxyliques avec les dérivés de formule (VII).

L'exemple suivant illustre l'invention, mais ne la limite en aucune façon.

**EXEMPLE : N-[(S) CARBETHOXY-1 BUTYL] (S) ALANINE**

**STADE A :** Ester benzylique de la (S) alanine, paratoluène sulfonate

Porter à reflux sous agitation une suspension de 8,2 kg de (S) alanine, 1,78 kg d'acide para toluène sulfonique, 2 kg d'alcool benzylique dans 3,30 litres de toluène, et distiller l'eau formée au fur et à mesure.

Le reflux est maintenu jusqu'à obtention de la quantité d'eau théorique.

Refroidir le milieu réactionnel à 70°C et l'évaporer sous vide. Le résidu pateux obtenu, couler 12,30 litres d'éther isopropylique et agiter 5 heures à 20-22°C.

La suspension obtenue est essorée et le précipité lavé par deux fois 1,50 litre d'éther isopropylique.

Après séchage, on obtient 32 kg du para toluène sulfonate de l'ester benzylique de la (S) alanine.

Rendement : 99,0%
Pouvoir rotatoire :

$$\alpha_D^{20} = -3°,7\ (c = 1/MeOH)$$

Point de fusion : 98°C
Spectrométrie dans l'infra-rouge (nujol) :
Bandes à 1760, 1730 $cm^{-1}$
Spectrométrie de résonance magnétique nucléaire $^1H$-60 MHz :
Solvant : Méthanol $D_4$

| | |
|---|---|
| δ=1,41 ppm, | doublet, 3H, CH-$\underline{CH_3}$, J=6,9 Hz |
| δ=2,29 ppm, | singulet, 3H, $\underline{CH_3}$-$C_6H_4$ |
| δ=4,02 ppm, | quadruplet, 1H, $\underline{CH}$-$CH_3$, J=6,9 Hz |
| δ=4,85 ppm, | signal large, 3H, $\underline{NH_2}$, $SO_3$ $\underline{H}$ |
| δ=5,21 ppm, | singulet, 2H, COO-$\underline{CH_2}$ |
| δ=7,0 à 7,8 ppm, | massif, 9H, aromatiques. |

**STADE B :** Ester benzylique de la S alanine

A une solution de 3,14 kg de para toluène sulfonate de l'ester benzylique de la (S) alanine dans 9,30 litres d'eau, ajouter jusqu'à alcalinisation de l'ammoniaque.

Maintenir trente minutes sous agitation, puis extraire par 1,25 litre de chlorure de méthylène. Décanter la phase organique et contre extraire la phase aqueuse par deux fois 1 litre de chlorure de méthylène.

Réunir les phases chlorométhyléniques, les laver à l'eau, puis sécher sur sulfate de magnésium.

Après évaporation du chlorure de méthylène, on obtient 1,5 kg d'ester benzylique de la (S) alanine sous forme d'un liquide huileux jaune.

Rendement : 95%
Spectrométrie dans l'infra-rouge (nujol) :
Bandes à 1760 et 1730 $cm^{-1}$.
Spectrométrie de résonance magnétique nucléaire $^1H$-60 MHz :

| | |
|---|---|
| δ=1,39 ppm, | doublet, 3H, CH-$\underline{CH_3}$, J=6,9 Hz |
| δ=3,98 ppm, | quadruplet, 1H, $\underline{CH}$-$CH_3$, J=6,9 Hz |
| δ=4,90 ppm, | signal large, 2H, $\underline{NH_2}$ |
| δ=5,23 ppm, | singulet, 2H, COO-$\underline{CH_2}$ |
| δ=7,10 ppm, | massif, 5H, aromatiques. |

**STADE C :** Ester benzylique de la N-[carbéthoxy-1 butyl] (S) alanine

Porter progessivement à 90°C, sous agitation, un mélange de 1,43 kg de l'ester benzylique de la (S) alanine, 184 kg d'α bromovalérate d'éthyle et 8,9 kg de triéthylamine dans 12 litres de N,N-diméthylformamide. Maintenir trois heures sous agitation à 90°C avant de refroidir le milieu réactionnel à 70°C.

Evaporer le N,N-diméthylformamide sous vide, puis reprendre le résidu huileux dans 7 litres d'éther isopropylique. Rajouter 7 litres d'eau et maintenir une demi heure sous agitation. Décanter la phase organique et extraire la phase aqueuse par 3,5 litres d'éther isopropylique. Joindre les phases organiques et les extraire par 9 litres d'acide chlorhydrique en solution molaire. Alcaliniser la phase aqueuse ainsi obtenue par du carbonate de sodium, puis extraire par de l'éther isopropylique. Réunir les phases organiques et les laver à l'eau; sécher sur sulfate de magnésium, puis évaporer sous vide.

On obtient 2,11 kg de l'ester benzylique de la N-[carbéthoxy-1 butyl] (S) alanine sous forme d'une huile légèrement colorée.

Rendement : 86%
Spectrométrie dans l'infra-rouge :
bande à 1760 et $1730^{-1}$.

Résonance magnétique nucléaire $^1$H-60 MHz :
Solvant $CCl_4$

| | |
|---|---|
| δ=0,65-1,60 ppm, | massif 13 H (2 × $CH_3$ + $C_3H_7$) |
| δ=2,11 ppm, | singulet, NH |
| δ=2,95 à 3,49 ppm, | multiplet, 2 × CH |
| δ=3,99 ppm, | quadruplet, 2H, $CH_2$-$CH_3$, J = 6,6 Hz |
| δ=5,02 ppm, | singulet, 2H, $CH_2$-O |
| δ=7,20 ppm, | massif, 5H, aromatiques. |

**STADE D :** Maléate de l'ester benzylique de la N-[(S) carbéthoxy-1 butyl] (S) alanine

A une solution de 1,9 kg de l'ester benzylique de la N-[carbéthoxy-1 butyl] (S) alanine dans un mélange de 10,44 litres de cyclohexane et 5,22 litres d'acétone, ajouter 376 g d'acide maléique. Le milieu est ensuite chauffé à 80°C sous agitation et la solution obtenue maintenue une heure à reflux. Laisser refroidir à température ambiante et maintenir douze heures sous agitation avant de glacer deux heures à 0-5°C.

Essorer le précipité et le laver sur filtre par du cyclohexane.

Après séchage, on obtient 1070 g de sel brut, qui sont recristallisés dans un mélange de cyclohexane et d'acétone.

On obtient ainsi 955 g de maléate de l'ester benzylique de la N-[(S) carbéthoxy-1 butyl] (S) alanine sous forme d'une poudre cristallisée blanche.

Pouvoir rotatoire :

$$\alpha_D^{20} = -19,0° \ (c = 1 / EtOH)$$

Point de fusion : 102°C
Spectrométrie dans l'infra-rouge (nujol) :
une bande à 1740 $cm^{-1}$
Résonance magnétique nucléaire $^1$H-60 MHz :
Solvant $CDCl_3$

| | |
|---|---|
| δ=0,80-2,10 ppm, | massif 13 H (2 × $CH_3$ + $C_3H_7$) |
| δ=3,89 ppm, | massif, 2H, 2CH |
| δ=4,19 ppm, | quadruplet, 2H, $CH_2$-$CH_3$, J = 6,8 H2 |
| δ=5,15 ppm, | singulet, 2H, $C_6H_5$-$CH_2$ |
| δ=5,89 ppm, | singulet, 3H, 2 × COOH + NH |
| δ=6,15 ppm, | singulet, 2H, CH = CH |
| δ=7,31 ppm, | singulet, 5H, aromatiques. |

**STADE E :** Ester benzylique de la N-[(S) carbéthoxy-1 butyl] (S) alanine

A une suspension de 0,72 kg de maléate de l'ester benzylique de la N-[(S) carbéthoxy-1 butyl] (S) alanine dans 13,50 litres d'eau, on additionne goutte à goutte jusqu'à alcalinisation de l'ammoniaque en maintenant la température inférieure à 20°C.

Décanter l'huile formée, extraire la phase aqueuse par quatre fois 2,25 litres d'éther isopropylique. Réunir les phases organiques et les sécher sur sulfate de magnésium avant de les évaporer sous vide.

On obtient 500 g de l'ester benzylique de la N-[(S) carbéthoxy butyl] (S) alanine, sous forme d'un liquide légèrement jaune.

Rendement : 95,5%
Pouvoir rotatoire :

$$\alpha_D^{20} = -48°,2 \ (c = 1 / EtOH)$$

Spectrométrie dans l'infra-rouge (nujol) :

Une bande à 1730 $cm^{-1}$.

Spectrométrie de résonance magnétique nucléaire $^1$H-60 MHz

Solvant : $CCl_4$

| | |
|---|---|
| δ=0,65-1,60 ppm, | massif 13 H (2 × $CH_3$ + $C_3H_7$) |
| δ=2,11 ppm, | singulet, NH |
| δ=2,95-3,49 ppm, | multiplet, 2 × CH |
| δ=3,99 ppm, | quadruplet, 2H, $CH_2$-$CH_3$, J = 6,6 Hz |
| δ=5,02 ppm, | singulet, (2H), $CH_2$-O |
| δ=7,2 ppm, | massif, 5H, aromatiques |

**Stade F :** N-[(S) carbéthoxy-1 butyl] (S) alanine

Dissoudre 4,5 kg de l'ester benzylique de la N-[(S) carbéthoxy-1 butyl] (S) alanine dans 75 litres d'éthanol, rajouter le catalyseur au palladium sur charbon à 5%, puis hydrogéner à pression atmosphérique à 20-22°C.

Diluer alors le milieu à l'éthanol et chauffer à 70°C pour passer en solution ; filtrer la solution chaude et rincer le catalyseur par 10 litres d'éthanol chaud.

Evaporer le solvant sous vide et cristalliser le résidu obtenu dans l'acétonitrile.

On obtient 2,61 kg de N-[(S) carbéthoxy-1 butyl] (S) alanine sous forme d'une poudre blanche.

Rendement : 82%

Pouvoir rotatoire :

$$\alpha_D^{20} = +4°,7 \ (c = 1 /H2O)$$

Point de fusion : 149°C

Analyse élémentaire : $C_{10}H_{19}NO_4$

| Elément | Théorie | Résultat |
|---|---|---|
| C | 55,28 | 55,28 |
| H | 8,81 | 8,97 |
| N | 6,45 | 6,35 |

Chromatographie sur couche mince :

Solvant : chloroforme : 70
méthanol : 30
acide acétique : 1

Rf : 0,45

Spectrométrie dans l'infra-rouge :

Bandes à 3050, 2250, 1740, 1620 et 1205 $cm^{-1}$

Spectrométrie de résonance magnétique nucléaire $^1$H-200 MHz :

Solvant DMSO $D_6$

| | |
|---|---|
| δ=0,86 ppm, | triplet, 3H, $(CH_2)_2$-$CH_3$ |
| δ=1,17 ppm, | doublet, 3H, CH-$CH_3$ |
| δ=1,18 ppm, | triplet, 3H, COO-$CH_2$-$CH_3$ |
| δ=1,30 ppm, | multiplet, 2H, CH2, $CH_2$-$CH_3$ |
| δ=1,50 ppm, | multiplet, 2H, $CH_2$-$CH_2$-$CH_3$ |
| δ=3,17 ppm, | quadruplet, 1H, CH, $CH_3$ |
| δ=3,26 ppm, | triplet, 1H, CH-$C_3H_7$ |
| δ=4,10 ppm, | quadruplet, 2H, COO-$CH_7$-$CH_3$ |
| δ=5,6 à 7,7 ppm, | massif, 2H échangeables, COOH et NH |

<u>Spectrométrie de résonance magnétique nucléaire</u> $^{13}C$-50 MHz :
Solvant DMSO $D_6$
173,9 et 175,4 signaux correspondant aux carboxyles.

## Revendications

1. Procédé de synthèse industrielle d'alpha amino diacides N-alkylés ou de leurs esters de formule (I) : dans laquelle

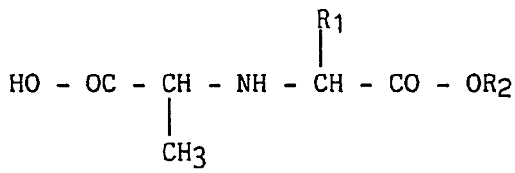

(I)

R1 représente un groupement alcoyle inférieur linéaire ou ramifié de 1 à 6 atomes de carbone,
R2 représente un groupement alcoyle inférieur linéaire ou ramifié de 1 à 4 atomes de carbone,
présentant la particularité d'avoir les deux carbones asymétriques ayant tous deux la configuration S (appelés dérivés de formule (IS)) caractérisé en ce que l'on, protège la L-Alanine de formule (IV) :

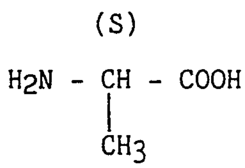

(IV)

dans laquelle le carbone asymétrique a la configuration S,
par estérification par l'alcool benzylique, en présence d'acide paratoluène sulfonique en distillant l'eau formée au fur et à mesure, pour obtenir un dérivé de formule (V) :

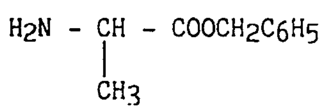

(V)

sous forme de paratoluène sulfonate que l'on transforme en la base correspondante par alcalinisation par l'ammoniaque qui, après extraction, est condensée, en présence d'une amine tertiaire avec un dérivé bromé de formule (VI) :

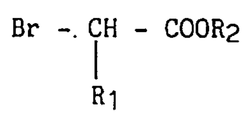

(VI)

dans laquelle, $R_1$ et $R_2$ ont la même signification que dans la formule (I), pour obtenir un dérivé de formule (VII):

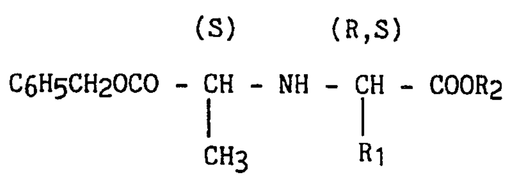

(VII)

dans laquelle, $R_1$ et $R_2$ ont la même signification que dans la formule (I),
dont on isole l'isomère (S, S) par mise en solution organique et addition à l'acide maléique,
le précipité obtenu étant filtré, séché et si on le désire, recristallisé,

pour donner un sel de maléate choisi et de l'isomère (S, S) du dérivé de formule (VII),
celui-ci étant libéré de son sel par alcalinisation d'une solution aqueuse suivie d'extraction et évaporation du solvant.
l'isomère (S, S) ainsi obtenu étant soumis à hydrogénation en présence de charbon palladié pour donner le composé de formule (I).

2. Procédé industriel selon la revendication 1 de synthèse industrielle d'un composé de formule (I), caractérisé en ce que la séparation de l'isomère (S, S) du mélange des deux isomères (S, R) et (S, S) du dérivé de formule (VII) obtenu après condensation du dérivé de formule (V) avec un dérivé de formule (VI),est réalisée par'addition d'acide maléique à une solution organique du mélange des deux isomères (S, R) et (S, S).

3. Procédé industriel de séparation de l'isomère (S, S) d'un mélange de deux isomères (R, S) et (S, S) de dérivés de formule (VII) selon la revendication 1, caractérisé en ce qu'une solution organique d'un mélange des deux isomères (S, S) et (S, R) est additionnée d'acide maléique, le précipité obtenu étant filtré, séché et recristallisé pour donner un sel de l'acide choisi et de l'isomère (S, S) du dérivé de formule (VII), celui-ci étant libéré de son sel par alcalinisation de sa solution aqueuse suivie d'extraction par un solvant convenable et de séparation du solvant.

4. Procédé industriel de synthèse selon l'une quelconque des revendications 1 à 3 d'un dérivé de formule (Ia), cas particulier de dérivé de formule (I) :
dans lequel $R_1$ est un groupement propyle,
$R_2$ est un groupement éthyle

```
HOOC - CH - NH - CH - COOC2H5
       |         |                      (Ia)
       CH3       CH2CH2CH3
```

présentant la particularité d'avoir les deux carbones asymétriques de configuration S (IaS), caractérisé en ce que le dérivé de formule (IV) selon la revendication 1 est condensé après protection de la fonction acide carboxylique par estérification par l'alcool benzylique en présence d'acide para toluène sulfonique avec l'α bromovalérate d'éthyle de formule (VIa) :

```
              (S)        (R,S)
C6H5CH2OCO - CH - NH - CH - COOC2H5        (VIIa)
             |         |
             CH3       CH2CH2CH3
```

pour obtenir le dérivé de formule (VIIa)

```
              (S)        (R,S)
C6H5CH2OCO - CH - NH - CH - COOC2H5        (VIIa)
             |         |
             CH3       CH2CH2CH3
```

dont on isole l'isomère (S, S) par mise en solution organique suivie d'addition d'acide maléique,
le précipité obtenu étant filtré, séché et recristallisé pour donner un sel de maléate et de l'isomère (S, S) du dérivé de formule (VIIa),
celui-ci étant libéré de son sel par alcalinisation d'une solution aqueuse suivie d'extraction par un solvant organique convenable et d'évaporation du solvant, l'isomère (S, S) ainsi obtenu étant soumis à hydrogénation catalytique pour donner le composé de formule (Ia).

5. Sel de l'isomère (S, S) du dérivé de formule (VIIa) avec l'acide maléique.

6. Utilisation d'un dérivé de formule (I) ou (Ia) obtenu selon l'une quelconque des revendications 1 à 4, pour la synthèse de dérivés de formule (II) :

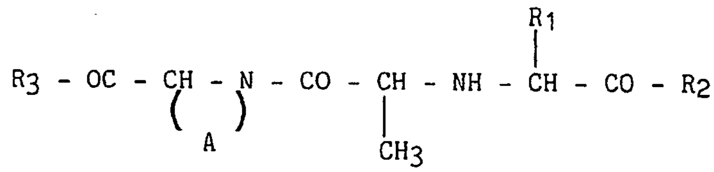 (II)

et leurs sels pharmaceutiquement acceptables,
dans laquelle

- $R_1$ et $R_2$ ont la même signification que dans la formule (I) ou (Ia),
- $R_3$ est atome d'hydrogène ou un groupement alcoyle de 1 à 4 atomes de carbone,
- la structure

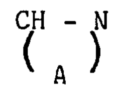

représente l'indoline, l'isoindoline, la tétrahydroquinoléine, la tétrahydroisoquinoléine, le perhydroindole, le perhydroisoindole, la perhydroisoquinoléine, la perhydroquinoléine, le perhydrocyclopent [b] pyrrole, l'aza-2-bicyclo [2,2,2] octane, l'aza-2 bicyclo [2,2,1] heptane.

7. Utilisation selon la revendication 6 du dérivé de formule (Ia) obtenu selon la revendication 4 dans la synthèse de dérivés de formule (II).

8. Utilisation selon l'une quelconque des revendications 6 ou 7 du dérivé de formule (Ia) dans la synthèse de dérivés de formule (II) dans laquelle

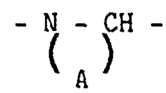

représente le perhydroindole.

9. Utilisation selon l'une quelconque des revendications 6 à 8 du dérivé de formule (Ia) dans la synthèse de l acide {[(éthoxycarbonyl)-1 butylamino-(S)]-2 propionyl-(S)}-1 octahydroindole carboxylique-2 (2S, 3aS, 7aS), ou perindopril.

## Ansprüche

1. Verfahren zur technischen Herstellung von N-alkylierten alpha-Aminosäuren oder deren Ester der Formel (I)

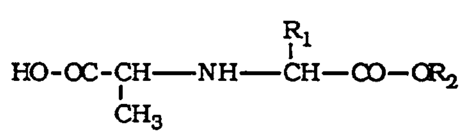 (I)

in der

$R_1$ eine geradkettige oderverzweigte niedrigmolekulare Alkylgruppe mit 1 bis 6 Kohlenstoffatomen und
$R_2$ eine geradkettige oder verzweigte niedrigmolekulare Alkylgruppe mit 1 bis 4 Kohlenstoffatomen bedeuten, mit der weiteren Maßgabe, daß die beiden asymmetrischen Kohlenstoffatome jeweils in der S-Konfiguration vorliegen (die auch als Derivate der Formel (IS) bezeichent werden), **dadurch gekennzeichnet,** daß man L-Alanin der Formel (IV)

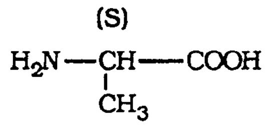 (IV)

in der das asymmetrische Kohlenstoffatom in der S-Konfiguration vorliegt, durch Verestern mit Benzylalkohol in Gegenwart von p-Toluolsulfonsäure und durch Abdestillieren des im Verlaufe der Reaktion gebildeten Wassers schützt unter Bildung eines Derivats der Formel (V)

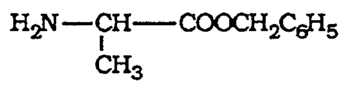 (V)

in Form des p-Toluolsulfonats, welches man durch Alkalischstellen mit Ammononiak in die entsprechende Base umwandelt, die nach der Extraktion in Gegenwart eines tertiären Amins mit einem Bromderivat der Formel (VI)

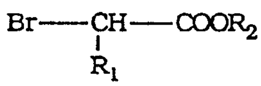 (VI)

in der $R_1$ und $R_2$ die bezüglich der Formel (I) angegebenen Bedeutungen besitzen, kondensiert wird zur Bildung eines Derivats der Formel (VII)

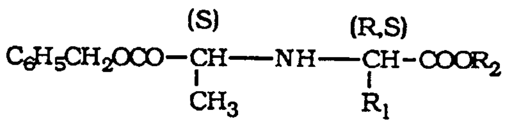 (VII)

in der $R_1$ und $R_2$ die bezüglich der Formel (I) angegebenen Bedeutungen besitzen, dessen (S, S)-Isomeres man durch Lösen in einem organischen Lösungsmittel und Zugabe von Maleinsäure isoliert, wobei der erhaltene Niederschlag abfiltriert, getrocknet und gewünschtenfalls umkristallisiert wird, so daß man das gewünschte Maleatsalz des (S, S)-Isomeren des Derivats der Formel (VII) erhält, welches durch Alkalischstellen einer wässrigen Lösung gefolgt von einer Extraktion und dem Verdampfen des Lösungsmittels aus seinem Salz freigesetzt wird, worauf das in dieser Weise erhaltene (S, S)-Isomere in Gegenwart von Palladium enthaltendem Kohlenstoff hydriert wird und zur Bildung der Verbindung der Formel (I).

2. Verfahren nach Anspruch 1 zur technischen Herstellung einer Verbindung der Formel (I), **dadurch gekennzeichnet,** daß man die Abtrennung des (S, S)-Isomeren aus der nach der Kondensation des Derivats der Formel (V) mit dem Derivat der Formel (VI) erhaltenen Mischung der beiden Isomeren (S, R) und (S, S) des Derivats der Formel (VII) durch Zugabe von Maleinsäure zu einer organischen Lösung der Mischung der beiden Isomeren (S, R) und (S, S) bewirkt.

3. Verfahren nach Anspruch 1 zur technischen Abtrennung des (S, S)-Isomeren aus einer Mischung der beiden Isomeren (R, S) und (S, S) der Derivate der Formel (VII), **dadurch gekennzeichnet,** daß man zu einer organischen Lösung einer Mischung der beiden Isomeren (S, S) und (S, R) Maleinsäure zusetzt, den erhaltenen Niederschlag abfiltriert, trocknet und umkristallisiert unter Bildung eines Salzes der gewählten Säure mit den (S, S)-Isomeren des Derivats der Formel (VII), welches durch Alkalischstellen seiner wässrigen Lösung gefolgt von einer Extraktion mit einem geeigneten Lösungsmittel und Abtrennung des Lösungsmittels aus seinem Salz freigesetzt wird.

4. Verfahren nach den Ansprüchen 1 bis 3 zur technischen Herstellung eines Derivats der Formel (Ia), als spezielles Derivat der Formel (I)

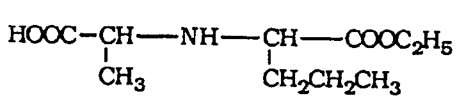 (Ia)

in der $R_1$ eine Propylgruppe und $R_2$ eine Ethylgruppe bedeuten, mit der Maßgabe, daß die beiden asymmetrischen Kohlenstoffatome in der S-Konfiguration vorliegen (IaS), **dadurch gekennzeichnet,** daß das Derivat der Formel (IV) nach Anspruch 1 nach dem Schützen der Carbonsäurefunktion durch Verestern mit Benzylalkohol

in Gegenwart von p-Toluolsulfonsäure mit α-Bromvaleriansäureethylester der Formel (VIa)

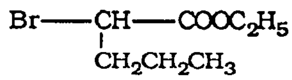 (VIa)

kondensiert wird zur Bildung eines Derivats der Formel (VIIa)

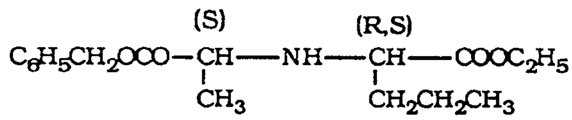 (VIIa)

dessen (S, S)-Isomeres man durch Lösen in einem organischen Lösungsmittel gefolgt von der Zugabe von Maleinsäure isoliert, worauf der erhaltene Niederschlag abfiltriert, getrocknet und umkristallisiert wird unter Erhalt eines Maleatsalzes des (S, S)-Isomeren des Derivats der Formel (VIIa), welches durch Alkalischstellen einer wässrigen Lösung gefolgt von einer Extraktion mit einem geeigneten organischen Lösungsmittel und Verdampfen des Lösungsmittels aus seinem Salz freigesetzt wird,
worauf das in diese Weise erhaltene (S, S)-Isomere einer katalytischen Hydrierung unterworfen wird zur Bildung der Verbindung der Formel (Ia).

5. Salz des (S, S)-Isomeren des Derivats der Formel (VIIa) mit Maleinsäure.

6. Verwendung eines Derivats der Formel (I) oder (Ia) erhalten nach einem der Ansprüche 1 bis 4 für die Herstellung von Derivaten der Formel (II)

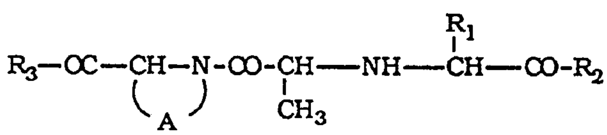 (II)

und deren pharmazeutisch annehmbaren Salze, worin

$R_1$ und $R_2$ die bezüglich der Formel (I) oder (Ia) angegebenen Bedeutungen besitzen,
$R_3$ ein Wasserstoffatom oder eine Alkylgruppe mit 1 bis 4 Kohlenstoffatomen darstellt und
die Gruppe der Formel

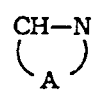

für Indolin, Isoindolin, Tetrahydrochinolin, Tetrahydroisochinolin, Perhydroindol, Perhydroisoindol, Perhydroisochinolin, Perhydrochinolin, Perhydrocyclopenta [b] pyrrol, 2-Aza-bicyclo [2.2.2] octan oder 2-Aza-bicyclo [2.2.1] heptan steht.

7. Verwendung nach Anspruch 6 des Derivats der Formel (Ia), erhalten nach Anspruch 4 bei der Herstellung der Derivate der Formel (VII).

8. Verwendung nach einem der Ansprüche 6 oder 7 des Derivats der Formel (Ia) für die Herstellung der Derivate der Formel (II), in der die Gruppe der Formel

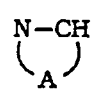

für Perhydroindol steht.

9. Verwendung nach einem der Ansprüche 6 bis 8 des Derivats der Formel (Ia) für die Herstellung von 1-{2-(1-(Ethoxycarbonyl)-(S)-butylamino]-(S)-propionyl}octahydroindol-2(2S, 3aS, 7aS)-carbonsäure oder Perindopril.

## Claims

1. Process for the industrial synthesis of N-alkylated alpha-amino diacids or their esters of formula (I) :

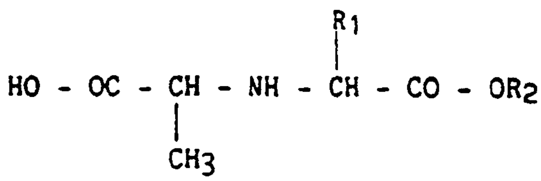

(I)

in which :

$R_1$ denotes a linear or branched lower alkyl group having from 1 to 6 carbon atoms,

$R_2$ denotes a linear or branched lower alkyl group having from 1 to 4 carbon atoms,

exhibiting the characteristic of having the two asymmetric carbon atoms both having the S configuration (referred to as derivatives of formula (IS)), characterised in that L-alanine of formula (IV) :

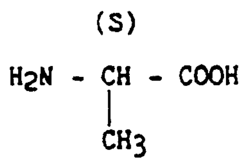

(IV)

in which the asymmetric carbon has the S configuration, is protected by esterification with benzyl alcohol, in the presence of para-toluenesulphonic acid with the water formed being progressively distilled off, to obtain a derivative of formula (V) :

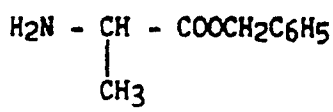

(V)

in the form of para-toluenesulphonate which is converted, by rendering alkaline with ammonia, into the corresponding base which, after extraction, is condensed, in the presence of a tertiary amine, with a brominated derivative of formula (VI) :

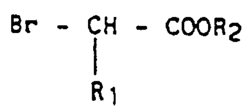

(VI)

in which $R_1$ and $R_2$ have the same meaning as in formula (I), to obtain a derivative of formula (VII) :

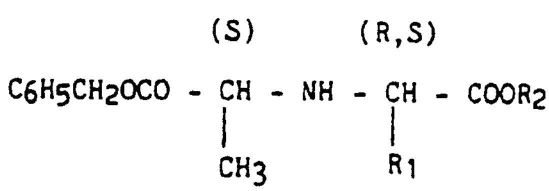

(VII)

in which $R_1$ 1 and $R_2$ have the same meaning as in formula (I),

the (S, S) isomer of which is isolated by bringing into organic solution and adding to maleic acid,

the precipitate obtained being filtered off, dried and, if desired, recrystallised,

to give a salt of the chosen maleate and of the (S, S) isomer of the derivative of formula (VII),

the latter being liberated from its salt by rendering an aqueous solution alkaline followed by extraction and evaporation of the solvent,

the (S, S) isomer thus obtained being subjected to hydrogenation in the presence of palladium/carbon to

give the compound of formula (I).

2. Industrial process according to claim 1 for the industrial synthesis of a compound of formula (I), characterised in that the separation of the (S, S) isomer from the mixture of the two isomers (S, R) and (S, S) of the derivative of formula (VII) obtained by condensing the derivative of formula (V) with a derivative of formula (VI) is effected by adding maleic acid to an organic solution of the mixture of the two isomers (S, R) and (S, S).

3. Industrial process for the separation of the (S, S) isomer from a mixture of the two isomers (R, S) and (S, S) of derivatives of formula (VII) according to claim 1, characterised in that maleic acid is added to an organic solution of a mixture of the two isomers (S, S) and (S, R), the precipitate obtained being filtered off, dried and recrystallised to give a salt of the chosen acid and of the (S, S) isomer of the derivative of formula (VIII), the latter being liberated from its salt by rendering alkaline its aqueous solution, followed by extraction with a suitable solvent and separation of the solvent.

4. Industrial synthesis process according to any one of claims 1 to 3 of a derivative of formula (Ia), that isin particular of a derivative of formula (I) :
in which $R_1$ is a propyl group,
$R_2$ is an ethyl group

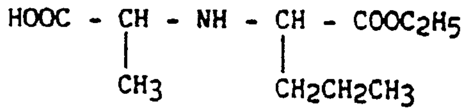

(Ia)

exhibiting the characteristic of having the two asymmetric carbons in the S configuration (IaS), characterised in that the derivative of formula (IV) according to claim 1 is condensed, after protection of the carboxylic acid function by esterification with benzyl alcohol in the presence of para-toluenesulphonic acid, with ethyl α-bromovalerate of formula (VIa) :

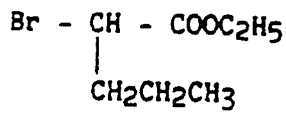

(VIa)

to obtain the derivative of formula (VIIa) :

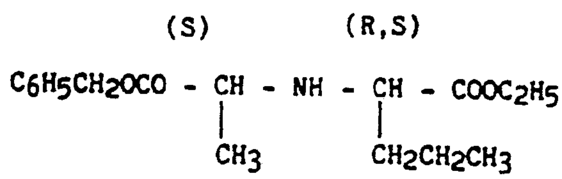

(VIIa)

the (S, S) isomer of which is isolated by bringing into organic solution, followed by the addition of maleic acid,
the precipitate obtained being filtered off, dried and recrystallised to give a salt of the maleate and of the (S, S) isomer of the derivative of formula (VIIa),
the latter being liberated from its salt by rendering an aqueous solution alkaline followed by extraction with a suitable organic solvent and evaporation of the solvent,
the (S, S) isomer thus obtained being subjected to catalytic hydrogenation to give the compound of formula (Ia).

5. Salt of the (S, S) isomer of the derivative of formula (VIIa) with maleic acid.

6. The use of a derivative of formula (I) or (Ia) obtained according to any one of claims 1 to 4 for the synthesis of derivatives of formula (II) :

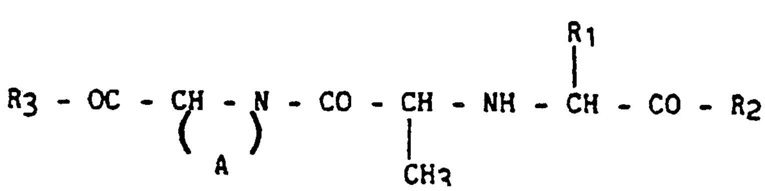

(II)

and their pharmaceutically acceptable salts,
in which :

- $R_1$ and $R_2$ have the same meaning as in formula (I) or (Ia),
- $R_3$ is a hydrogen atom or an alkyl group having from 1 to 4 carbon atoms,
- the structure

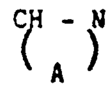

denotes indoline, isoindoline, tetrahydroquinoline, tetrahydroisoquinoline, perhydroindole, perhydroisoindole, perhydroisoquinoline, perhydroquinoline, perhydrocyclopenta [b] pyrrole, 2-azabicyclo [2,2,2]octane or 2-azabicyclo [2,2,1] heptane.

7. Use according to claim 6 of the derivative of formula (Ia) obtained according to claim 4 in the synthesis of derivatives of formula (II).

8. Use according to either of claims 6 and 7 of the derivative of formula (Ia) in the synthesis of derivatives of formula (II) in which

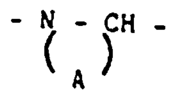

denotes perhydroindole.

9. Use according to any one of claims 6 to 8 of the derivative of formula (Ia) in the synthesis of (2S, 3aS, 7aS)-1-{2-[1-(ethoxycarbonyl)-(S)-butylamino]-(S)-propionyl}-octahydroindole-2-carboxylic acid, or perindopril.